# DEMANDE DE BREVET EUROPEEN

(11) **EP 1 138 266 A1**
(43) Date de publication de la demande: **04.10.2001**
(21) Numéro de dépôt: 01400649.8
(22) Date de dépôt: 13.03.2001
(51) Int. Cl.: A61B 17/58, A61B 17/86

(54) **Système permettant de maintenir deux portions d'os l'une par rapport à l'autre**

(30) Priorité: 24.03.2000 FR 0003757
(71) Demandeur: Renard, Xavier, 91430 Vauhallan (FR)
(72) Inventeur: Renard, Xavier, 91430 Vauhallan (FR)
(74) Mandataire: Flavenot, Bernard

(57) **Abrégé**

Le système selon l'invention se caractérise essentiellement par le fait qu'il comporte une broche 20 constituée d'une tige 22 comprenant une extrémité pénétrante 23 comportant un filetage 25 sur sa paroi latérale 21 et une extrémité de préhension 24 comportant un filetage 27 sur sa paroi latérale 26, les deux filetages 25, 27 ayant des sens de vissage opposés, d'une pièce écrou 28 comportant une percée traversante taraudée 29 apte à se visser sur le filetage 27, et un élément coupant à deux mâchoires 40, 41, l'extrémité coupante de chaque mâchoire étant en forme de biseau, le fond 43 du second filetage 27 formant sensiblement un dièdre d'angle au sommet égal à α et l'angle au sommet du biseau étant au plus égal à α.

Application, notamment, à la réduction des fractures, par exemple de métacarpiens, de trochanters, de radius, etc.

## Description

La présente invention concerne les systèmes pour réaliser le maintien de deux os, ou portions d'os, l'un par rapport à l'autre, qui trouvent des applications avantageuses pour la réduction des fractures, par exemple de métacarpiens, de trochanters, de radius, etc.

On connaît déjà des systèmes permettant de maintenir deux portions d'os A et B l'une par rapport à l'autre, par exemple des broches comme la broche 1 schématiquement illustrée sur la figure 1 intitulée "art antérieur". De telles broches sont par exemple décrites dans les DE-U-91 05 152 et JP-A-10 052439.

Cette broche 1 selon l'art antérieur comporte essentiellement une tige 2 comprenant une extrémité pénétrante 3 et une extrémité de préhension 4. L'extrémité pénétrante 3 comporte un premier filetage 5 qui peut, ou non, être autotaraudant. Ce filetage a une valeur de pas donnée qui permet à la tige 2 de s'ancrer dans l'une B des deux portions d'os. L'extrémité de préhension 4 comporte, sur sa paroi latérale 6, un second filetage 7 de même sens que le premier et d'un pas donné avec lequel peut coopérer un écrou 8 qui déborde radialement de la tige et assez largement de façon à constituer une surface d'appui.

Lorsque l'on veut maintenir deux portions d'os A et B accolées l'une à l'autre, par exemple pour réduire une fracture d'un métacarpien, à l'aide d'une broche telle que décrite ci-dessus, on peut commencer par réaliser, dans la première portion d'os A, une percée 9 d'un diamètre au moins égal au diamètre du premier filetage 5, cette percée 9 pouvant d'ailleurs être réalisée avec l'extrémité pénétrante de la tige elle-même lorsqu'elle est autotaraudante.

Après la réalisation de cette percée, on ancre l'extrémité pénétrante 3 de la tige 2 dans la seconde portion d'os B de façon que l'extrémité de préhension 4 émerge de la percée 9 par son ouverture 10 opposée à celle 11 qui est la plus proche de la seconde portion d'os B.

Ensuite, on visse l'écrou 8 sur la partie de tige qui émerge de la percée jusqu'à ce que la première portion d'os A soit prise en pincement entre la seconde portion d'os B et l'épaulement constitué par l'écrou 8. En vissant plus ou moins l'écrou 8 sur la partie de tige émergeant de la percée 9, on appuie les deux portions d'os A et B plus ou moins fortement l'une contre l'autre.

Cette broche donne de bons résultats mais présente des inconvénients. L'un de ces inconvénients consiste dans le fait que, pour obtenir un ajustement des deux portions d'os comme le souhaite le praticien, il faut, pendant l'opération de mise en place de la broche, maintenir les deux portions d'os l'une par rapport à l'autre et la tige tout en pivotant l'écrou. Un autre inconvénient consiste dans le fait que, lorsque l'écrou vient au contact de la première portion d'os A et que l'on continue à agir sur lui pour rapprocher les deux portions d'os, il porte sur la surface de la première portion d'os A sans pouvoir s'y ancrer. Il peut ainsi glisser au cours du temps sur cette portion d'os, entraînant un relâchement dans le maintien des deux portions d'os l'une par rapport à l'autre, résultat contraire à celui qui est recherché.

Pour tenter de pallier ces inconvénients il a été réalisé des systèmes comme celui qui est décrit dans le document US-A-5 409 486. Très schématiquement, ce système comporte une broche constituée d'une tige comprenant une extrémité pénétrante et une extrémité de préhension, la tige comportant, sur son extrémité pénétrante, une première partie présentant, sur sa surface latérale, un premier filetage et, sur son extrémité de préhension, une seconde partie présentant, sur sa surface latérale, un second filetage, les premier et second filetages ayant des sens de vissage opposés, et une pièce écrou comportant une percée traversante taraudée apte à se visser sur le second filetage.

Ce système donne de bons résultats mais présente encore des inconvénients. Notamment, il ne permet pas d'effectuer une coupe franche et nette de l'extrémité de préhension de la tige qui dépasse de l'écrou après que la broche ait été placée par exemple pour la réduction d'une fracture comme décrit ci-dessus, et il ne permet pas d'assurer la solidarisation de l'écrou avec la tige en évitant qu'il se dévisse dans le temps.

Aussi, la présente invention a-t-elle pour but de réaliser un système qui pallie au moins une grande partie des inconvénients mentionnés ci-dessus.

Plus précisément, la présente invention a pour objet un système pour réaliser le maintien de deux portions d'os l'une par rapport à l'autre, comportant :
- une broche constituée d'une tige comprenant une extrémité pénétrante comportant un premier filetage sur sa paroi latérale et une extrémité de préhension comportant un second filetage sur sa paroi latérale, les premier et second filetages ayant des sens de vissage opposés, et d'une pièce écrou comportant une percée traversante taraudée apte à se visser sur le second filetage, et
- un élément coupant à deux mâchoires, l'extrémité coupante de chaque mâchoire étant en forme de biseau,
caractérisé par le fait que le fond du second filetage forme sensiblement un dièdre d'angle au sommet égal à α, et que l'angle au sommet dudit biseau est au plus égal à α.

D'autres caractéristiques et avantages de l'invention apparaîtront au cours de la description suivante donnée en regard des dessins annexés à titre illustratif mais nullement limitatif, dans lesquels :
La figure 1 représente, dans une vue en perspective, un mode de réalisation de broche selon l'art antérieur tel que décrit ci-avant,
La figure 2 représente une coupe schématique d'un mode de réalisation du système selon l'invention pour maintenir deux portions d'os l'une par rapport à l'autre,
La figure 3 représente, vue en coupe et à plus grande échelle que celle des figures 1 et 2, une partie d'un mode de réalisation perfectionné du système selon l'invention,
La figure 4 représente une extrémité d'une partie du système selon l'invention dans sa configuration finale, dans laquelle il assure le maintien de deux portions d'os l'une par rapport à l'autre, et
Les figures 5 et 6 représentent deux vues en coupe d'une partie d'un autre mode de réalisation d'un système selon l'invention, la figure 5 étant une coupe référencée V-V sur la figure 6 et la figure 6 étant une coupe référencée VI-VI sur la figure 5.

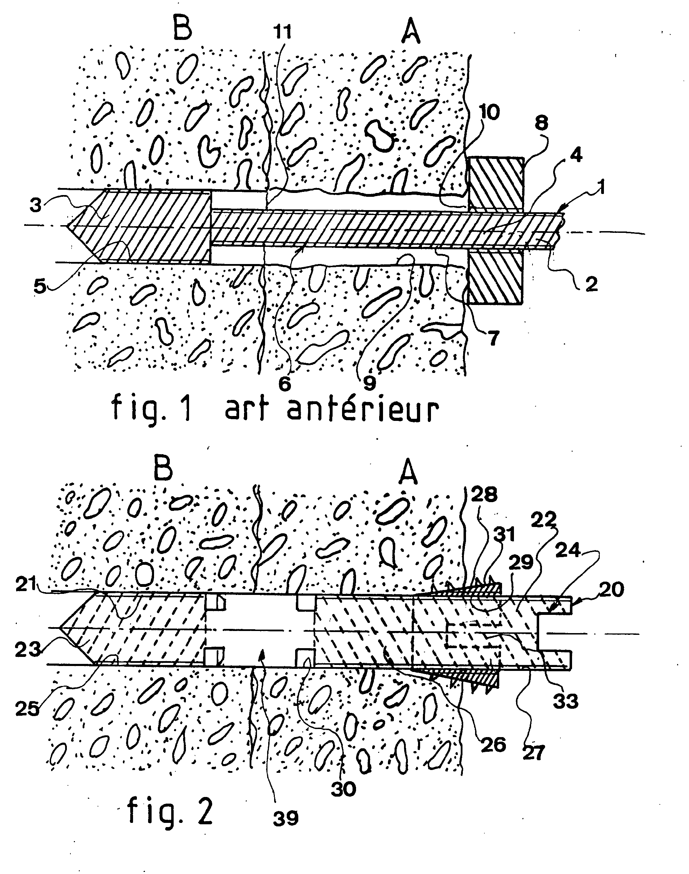

Le Demandeur tient à préciser que les figures 2 à 6 représentent trois modes de réalisation préférés de l'objet selon l'invention mais qu'il peut exister d'autres modes de réalisation qui répondent à la définition de cette invention. Cependant, dans le but de faciliter la compréhension de la présente description, les mêmes références y désignent les mêmes éléments, quelle que soit la figure sur laquelle elles apparaissent et quelle que soit la forme de représentation de ces éléments. De même, si des éléments ne sont pas spécifiquement référencés sur l'une des figures, leurs références peuvent être aisément retrouvées en se reportant à une autre figure.

II précise en outre que, lorsque, selon la définition de l'invention, l'objet de l'invention comporte "au moins un" élément ayant une fonction donnée, le mode de réalisation décrit peut comporter plusieurs de ces éléments.

Il précise aussi que, si le mode de réalisation de l'objet selon l'invention tel qu'illustré comporte plusieurs éléments de fonction identique et que si, dans la description, il n'est pas spécifié que l'objet selon cette invention doit obligatoirement comporter un nombre particulier de ces éléments, l'objet de l'invention pourra être défini comme comportant "au moins un" de ces éléments.

Le système selon l'invention pour maintenir deux portions d'os A et B l'une par rapport à l'autre comporte tout d'abord une broche 20 dont un mode de réalisation avantageux est schématiquement représenté sur la figure 2.

Cette broche 20 comporte une tige 22 comprenant une extrémité pénétrante 23 et une extrémité de préhension 24, et une pièce écrou 28. L'extrémité pénétrante 23 comporte, sur sa surface latérale 21, un premier filetage 25 et l'extrémité de préhension 24 comporte, sur sa surface latérale 26, un second filetage 27. La pièce écrou 28 comporte une percée traversante taraudée 29 apte à se visser sur le second filetage 27, les premier 25 et second 27 filetages ayant des sens de vissage opposés.

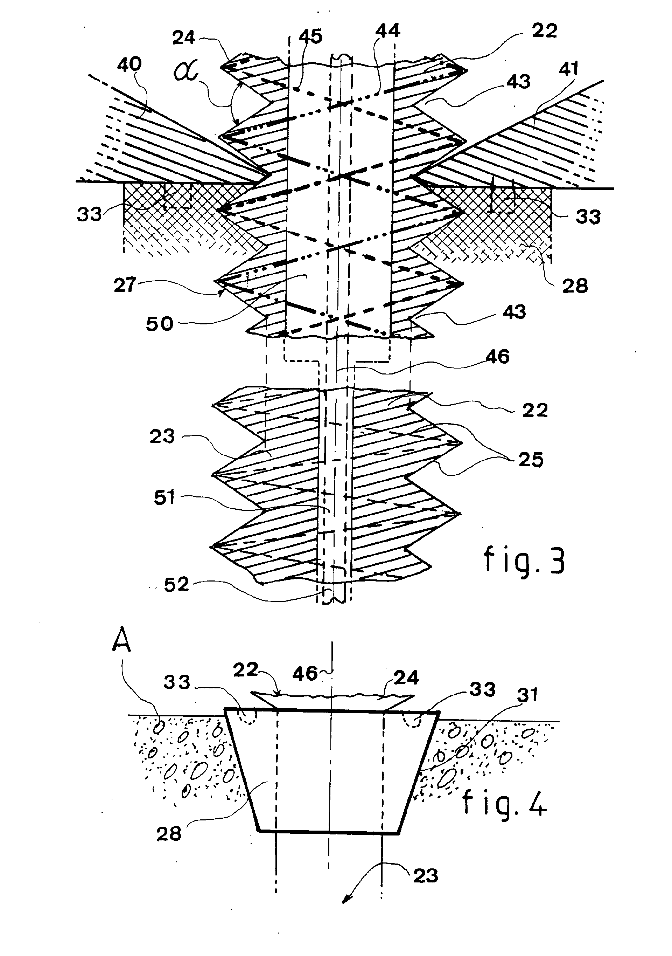

Comme illustré sur la figure 3, le système comporte en outre un élément coupant à deux mâchoires 40, 41, comme une pince ou analogue, l'extrémité coupante de chaque mâchoire étant en forme de biseau.

Selon une caractéristique de l'invention, le fond 43 du second filetage 27 forme sensiblement un dièdre d'angle au sommet égal à α et l'angle au sommet de la forme en biseau de l'extrémité coupante de chaque mâchoire 40, 41 est au plus égal à α, et même avantageusement légèrement inférieur, dans le but qui sera explicité ci-après. A titre d'exemple, l'angle α est égal à soixante degrés.

De plus, de façon préférentielle, le second filetage 27 est constitué de deux filetages secondaires dont les chemins sont illustrés sur la figure 3 par deux types différents de lignes interrompues 44, 45. Ces deux filetages secondaires sont de même pas, sensiblement de même profil, mais décalés d'un demi-pas l'un de l'autre, et le fond de ces deux filetages secondaires forme un dièdre d'angle au sommet égal à α.

Ainsi, par cette caractéristique, il existe, dans tout plan perpendiculaire à l'axe 46 de la tige, deux fonds de filets opposés situés sur la paroi latérale de l'extrémité de préhension 24 de la tige 22. De cette façon, les deux mâchoires 40, 41 peuvent toujours être positionnées pour obtenir une coupe parfaitement nette de l'extrémité de préhension 24 de la tige dans un plan perpendiculaire à l'axe 46, sans aspérité particulière dépassant de l'écrou, comme il sera explicité ci-après en regard de la figure 4.

En outre, dans une réalisation avantageuse comme celle illustrée sur la figure 2, la broche 20 comporte, entre les extrémités pénétrante 23 et de préhension 24 de la tige, des moyens 39 pour réaliser dans l'os un orifice à paroi sensiblement lisse d'une section au moins égale à la section de l'extrémité pénétrante 23 et définie par les sommets du premier filetage 25. Ces moyens 39 peuvent par exemple être en forme de gouge circulaire.

De cette façon, quand le premier filetage 25 est autotaraudant, au fur et à mesure que l'extrémité pénétrante 23 se visse dans l'os en s'y ancrant, les moyens 39 détruisent les filets qui ont été réalisés lors du passage du premier filetage autotaraudant et réalisent un orifice 30 à paroi relativement lisse d'un diamètre sensiblement égal à celui défini par les sommets de ce premier filetage.

Selon une réalisation préférentielle, la pièce écrou 28 présente une surface extérieure 31 de forme sensiblement tronconique de façon qu'elle puisse pénétrer entre la matière osseuse de la portion d'os A et la tige 22, et s'ancrer dans la matière osseuse.

Il est même avantageux que la surface extérieure 31 de la pièce écrou 28 comporte des moyens pour réaliser un meilleur ancrage de cette pièce écrou dans la portion d'os A, par exemple des picots ou analogues qui peuvent pénétrer dans l'os en s'y enfonçant au moins partiellement.

Il est bien précisé que, bien que les deux filetages 25, 27 soient en sens inverse l'un de l'autre, ils peuvent avoir des pas égaux ou différents. De même, le diamètre défini au sommet du premier filetage 25 peut être égal au diamètre défini au sommet du second filetage 27, ou différent. La figure 2 représente un mode de réalisation avantageux pour un bon maintien des deux portions d'os, dans lequel le diamètre défini au sommet des filets du premier filetage 25 est le même que celui défini au sommet des filets du second filetage 27.

Dans une réalisation possible, la pièce écrou 28 peut être constituée en au moins deux parties aptes à s'associer suivant un plan longitudinal passant par l'axe de la percée traversante taraudée. Elle peut aussi comporter des fentes longitudinales 33 aptes à recevoir l'extrémité d'un outil de vissage.

Bien entendu, la tige et la pièce écrou sont réalisées en un matériau métallique, par exemple un matériau à base de titane.

Dans un mode de réalisation perfectionné du système selon l'invention, comme celui représenté sur la figure 3, la tige 22 comporte une première percée axiale 50 dans au moins une partie de son extrémité de préhension 24, cette première percée axiale 50 ayant une section d'une première valeur donnée pour affaiblir la paroi de cette extrémité de préhension.

La tige 22 peut aussi comporter une seconde percée axiale 51 réalisée dans son extrémité pénétrante 23, cette seconde percée axiale 51 étant coaxiale à la première percée axiale 50 et en continuité de celle-ci, et ayant une section d'une seconde valeur au plus égale à celle de la première percée axiale, cette seconde valeur étant déterminée pour laisser passer à frottements doux, par exemple, une aiguille de guidage d'implantation représentée schématiquement en traits interrompus en 52. La fonction d'une telle aiguille de guidage d'implantation est bien connue des hommes du métier et ne sera donc pas décrite ici.

Le système décrit ci-dessus pour obtenir le maintien de deux portions d'os A et B l'une par rapport à l'autre s'utilise de la façon suivante :

Quand on veut rapprocher et maintenir accolés deux os ou deux portions d'os A et B, on utilise tout d'abord la tige 22 en la tournant de façon à faire pénétrer son extrémité pénétrante 23 dans la portion d'os A, puis dans la portion d'os B jusqu'à ce qu'elle y soit entièrement contenue, comme représenté sur la figure 2. Au cours de la translation en rotation de la tige 22, les moyens 39 avancent avec la tige et réalisent, dans l'os, à l'arrière de l'extrémité pénétrante 23, un orifice 30 à paroi lisse et d'un diamètre égal à celui défini au sommet des filets du filetage 25. L'extrémité de préhension 24 peut ainsi se déplacer sans contrainte dans la portion d'os A tout en étant ajustée dans cet orifice 30.

Lorsque la tige 22 est dans la position décrite ci-dessus, la pièce écrou 28 est mise en place sur l'extrémité de préhension 24 au contact de la portion d'os A et avec un début d'ancrage dans cette portion d'os. La pièce écrou est placée autour de l'extrémité de préhension 24 si elle est en deux parties, ou vissée au moyen d'un outil de vissage inséré dans les fentes 33, par exemple un tournevis creux qui se place autour de l'extrémité de préhension.

Ensuite, le praticien tourne la tige 22 par son extrémité de préhension 24, dans le même sens que celui qui permet à l'extrémité pénétrante d'avancer dans l'os B. Comme la pièce écrou 28 est vissée sur un filetage de sens inverse de celui qui permet à l'extrémité pénétrante d'avancer dans l'os B, cette pièce écrou avance dans la portion d'os A, sans pivoter par rapport à cette portion d'os. La pièce écrou vient donc automatiquement, par un mouvement de translation relatif, s'ancrer partiellement ou en totalité dans cette portion d'os A comme représenté sur la figure 2.

Pour associer les deux portions d'os A et B, il suffit de faire effectuer à la tige 22 une très faible rotation après que la pièce écrou soit arrivée au contact pénétrant de la portion d'os A. Si le praticien juge que l'effort exercé sur les deux portions d'os A et B est trop important, il peut faire pivoter la tige dans le sens contraire à celui du vissage de l'extrémité pénétrante, pour tendre à la faire ressortir.

Il faut enfin noter que, dès l'instant où la pièce écrou 28 est mise en place sur l'extrémité de préhension 24 et au contact ancrant de la portion d'os A, il suffit au praticien d'agir sur la tige 22 par cette extrémité de préhension pour ajuster l'intensité de l'effort de maintien des deux portions d'os, sans tenir la pièce écrou 28.

Quand le praticien estime que la broche 20 a pris sa place définitive, il coupe l'extrémité de préhension 24 au moyen des deux mâchoires 40, 41 de l'élément coupant du système comme décrit ci-avant, en plaçant les deux mâchoires le plus près possible de l'écrou 28 et dans deux creux opposés du double-filet 44, 45 comme illustré sur la figure 3.

Comme la paroi de l'extrémité de préhension 24 est affaiblie par la percée axiale 50, elle se coupe très facilement et, en plus, en s'ovalisant par aplatissement dans le plan défini par les deux extrémités en biseau des deux mâchoires coupantes. On obtient une coupe comme représenté schématiquement sur la figure 4. L'aplatissement de la paroi de l'extrémité de préhension au niveau de la coupe permet d'obtenir une sorte de sertissage de l'écrou 28, l'empêchant de se dévisser accidentellement dans le temps. Cependant, ce sertissage permet malgré tout de continuer le vissage de l'écrou pour par exemple l'enfouir totalement dans l'os et même, si cela est nécessaire, de le dévisser.

La figure 4 représente la tige 22 selon la figure 3 après la coupe de l'extrémité de préhension 24, vue dans un plan perpendiculaire à celui dans lequel est représentée la tige 22 sur cette figure 3.

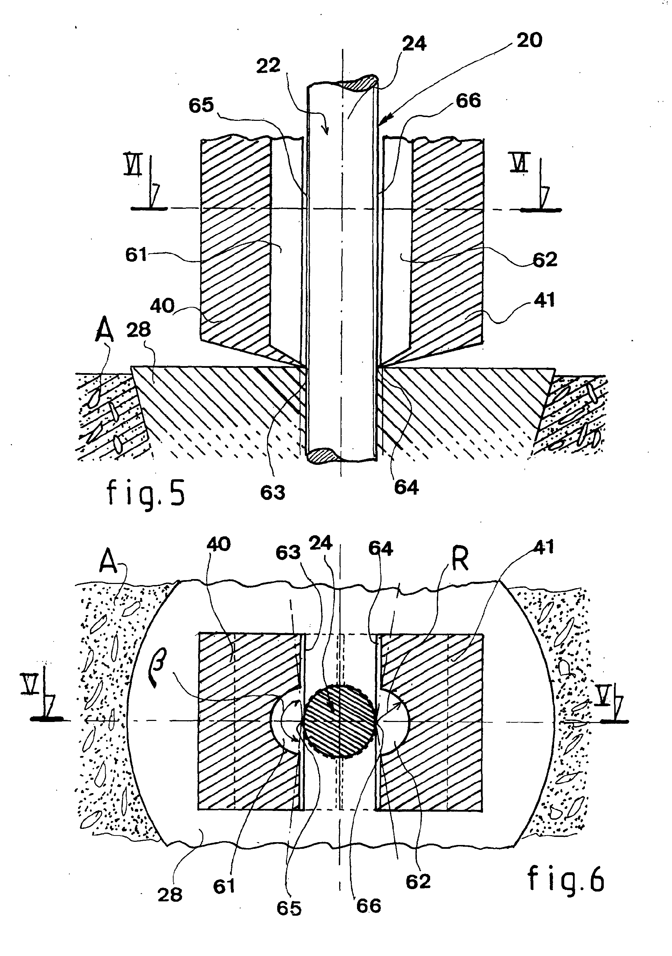

Selon le mode de réalisation illustré sur les figures 5 et 6, le système selon l'invention comporte, en plus des éléments décrits ci-avant, deux coquilles 61, 62 de forme sensiblement cylindrique de révolution, ces coquilles étant ouvertes sur un angle au centre β au plus égal à cent-quatre-vingts degrés. Sur ces figures, l'angle d'ouverture de ces coquilles est de l'ordre de cent-soixante-dix degrés. En outre, le rayon R de ces deux coquilles est sensiblement égal à celui de l'extrémité de préhension 24 de la tige 22.

Ces deux coquilles 61, 62 sont respectivement solidaires des deux mâchoires 40, 41 qui forment chacune, dans le mode de réalisation illustré, un biseau ayant une arête de coupe 63, 64 sensiblement rectiligne. Les deux coquilles sont solidaires respectivement des mâchoires de façon que leur axe de révolution 65, 66 soit sensiblement perpendiculaire aux arêtes des mâchoires et chaque axe de révolution est avantageusement concourant avec l'arête de la mâchoire correspondante.

En outre, ces coquilles sont solidarisées avec les deux mâchoires de façon que, lorsque les deux mâchoires sont au contact l'une de l'autre par leurs arêtes 63, 64, les coquilles viennent en regard l'une de l'autre pour délimiter un volume cylindrique de révolution, qui est fermé si l'angle d'ouverture de ces coquilles est de cent-quatre-vingts degrés, dont le diamètre est sensiblement identique à celui de l'extrémité de préhension 24 de la tige 22.

Ces deux coquilles sont en outre solidaires des deux mâchoires de façon que, lorsque les deux mâchoires viennent couper l'extrémité de préhension 24 comme explicité notamment en regard des figures 3 et 4, les deux coquilles viennent entourer la partie de cette extrémité de préhension qui doit être détachée et qui se situe au-dessus de l'écrou 28 par rapport à l'extrémité pénétrante 23.

Dans un mode de réalisation avantageux, comme illustré sur les figures, une mâchoire et la coquille solidaire de cette mâchoire seront réalisées en une seule pièce dans un même matériau, par exemple par forgeage.

Le mode de réalisation du système selon l'invention tel décrit ci-dessus présente incontestablement des avantages.

Notamment, il permet d'effectuer une coupe de l'extrémité de préhension 24 suivant un plan qui sera rigoureusement perpendiculaire à l'axe de la tige 22. Les deux mâchoires de l'élément coupant ne seront en priorité utilisées que sur leur partie centrale destinée à la coupe, est non pas par exemple sur leurs parties latérales, ce qui entraîne généralement, très rapidement, une déformation de l'élément de coupe.

Avec un élément coupant du système décrit ci-dessus, il sera difficile de couper des extrémités de préhension 24 ayant des diamètres plus importants que ceux pour lesquels cet élément coupant a été expressément prévu, contribuant ainsi à augmenter la durée de vie des mâchoires de cet élément coupant.

Enfin, l'un des avantages du système, notamment dans son application dans le domaine médical comme décrit ci-dessus est le suivant : lorsque, après avoir implanté une broche 20 pour réunir deux os comme décrit ci-dessus dans le but par exemple de la réduction d'une fracture, un praticien procède à la coupe de l'extrémité de préhension émergente 24, les deux mâchoires enserrent cette partie de tige et la retiennent lorsqu'elle est coupée, évitant qu'elle ne soit éjectée de façon aléatoire dans l'espace environnant et, surtout, qu'elle ne tombe dans le corps humain avec tous les inconvénients qui peuvent alors s'en suivre si elle n'est pas récupérée.

En outre, il est à remarquer que, avant de procéder à la coupe de l'extrémité de préhension 24, cette dernière émerge largement de la partie osseuse A. Aussi, de façon avantageuse, l'élément coupant comporte un guide (non-illustré) monté en coopération avec les deux mâchoires 40, 41, apte à entourer l'extrémité émergente 24 avant sa coupe, par exemple un manchon situé dans l'axe des deux coquilles lorsqu'elles sont associées pour former le volume cylindrique de révolution défini ciavant entourant l'extrémité de préhension.

De cette façon, le praticien peut positionner l'élément coupant en plaçant le manchon sur l'extrémité de préhension et faire glisser les deux mâchoires ouvertes 40, 41 jusqu'à ce qu'elles viennent au contact de l'écrou 28, figure 3, et procéder alors à la coupe de l'extrémité de préhension. Il est ainsi certain que les deux mâchoires seront automatiquement exactement situées à l'endroit voulu pour enserrer l'extrémité émergente et la couper. En quelque sorte, le praticien peut en toute sécurité effectuer la coupe de l'extrémité émergente 24 suivant la technique dite "à l'aveugle".

## Revendications

1. Système pour réaliser le maintien de deux portions d'os (A, B) l'une par rapport à l'autre, comportant :
- une broche (20) constituée d'une tige (22) comprenant une extrémité pénétrante (23) comportant un premier filetage (25) sur sa paroi latérale(21) et une extrémité de préhension (24) comportant un second filetage (27) sur sa paroi latérale (26), les premier et second filetages (25, 27) ayant des sens de vissage opposés, et d'une pièce écrou (28) comportant une percée traversante taraudée (29) apte à se visser sur le second filetage (27), et
- un élément coupant à deux mâchoires (40, 41), l'extrémité coupante de chaque mâchoire étant en forme de biseau,
**caractérisé par** le fait que le fond (43) du second filetage (27) forme sensiblement un dièdre d'angle au sommet égal à α, et que l'angle au sommet dudit biseau est au plus égal à α.

2. Système selon la revendication 1, **caractérisé par** le fait que ledit second filetage (27) est constitué de deux filetages secondaires (44, 45) de même pas, sensiblement de même profil et décalés d'un demi-pas, le fond des deux filetages secondaires formant un dièdre d'angle au sommet égal à α.

3. Système selon l'une des revendications 1 et 2, **caractérisé par** le fait que ladite tige (22) comporte une première percée axiale (50) dans au moins une partie de son extrémité de préhension (24), cette première percée axiale ayant une section d'une première valeur donnée pour affaiblir la paroi de ladite extrémité de préhension.

4. Système selon la revendication 3, **caractérisé par** le fait que ladite tige comporte une seconde percée axiale (51) réalisée dans son extrémité pénétrante (23), cettedite seconde percée axiale étant coaxiale à la première percée axiale et en continuité de celle-ci, la section de cette seconde percée axiale ayant une seconde valeur au plus égale à celle de la première percée axiale (50) déterminée pour laisser passer à frottements doux une aiguille de guidage d'implantation (52).

5. Système selon l'une des revendications 1 à 4, **caractérisé par** le fait que la tige (22) comporte, entre ses extrémités pénétrante (23) et de préhension (24), des moyens (39) pour réaliser, dans un os, un orifice (30) à paroi sensiblement lisse et d'une section au moins égale à la section de l'extrémité pénétrante (23) de la tige définie au sommet du premier filetage.

6. Système selon l'une des revendications 1 à 5, **caractérisé par** le fait que la section de l'extrémité pénétrante (23) de la tige définie au sommet du premier filetage (25) est égale à la section de l'extrémité de préhension (24) de la tige définie au sommet du second filetage.

7. Système selon l'une des revendications 1 à 6, **caractérisé par** le fait que ladite pièce écrou (28) est constituée par au moins deux parties aptes à s'associer suivant un plan longitudinal passant par l'axe de la percée traversante taraudée (29).

8. Système selon l'une des revendications 1 à 7, **caractérisé par** le fait que ladite pièce écrou (28) comporte des fentes longitudinales (33) aptes à recevoir l'extrémité d'un outil de vissage.

9. Système selon l'une des revendications 1 à 8, **caractérisé par** le fait que la tige et la pièce écrou sont réalisées en un matériau métallique à base de titane.

10. Système selon l'une des revendications 1 à 9, **caractérisé par** le fait qu'il comporte deux coquilles (61, 62) de forme sensiblement cylindrique de révolution, ces coquilles étant ouvertes sur un angle au centre (β) au plus égal à cent-quatre-vingts degrés et ayant un rayon (R) sensiblement égal à celui de l'extrémité de préhension (24), ces deux coquilles (61, 62) étant respectivement solidaires des deux mâchoires (40, 41) présentant un biseau d'arête de coupe (63, 64) sensiblement rectiligne de façon que leur axe de révolution (65, 66) soit sensiblement perpendiculaire aux arêtes de coupe et que chaque axe de révolution soit sensiblement concourant avec l'arête de la mâchoire correspondante, et que, lorsque les deux mâchoires sont au contact l'une de l'autre par leurs arêtes (63, 64), lesdites coquilles viennent en regard l'une de l'autre pour délimiter un volume cylindrique de révolution dont le diamètre est sensiblement identique à celui de ladite extrémité de préhension (24).

11. Système selon la revendication 10, **caractérisé par** le fait que les deux dites coquilles (61, 62) sont en outre solidaires des deux mâchoires (40, 41) de façon que, lorsque les deux mâchoires viennent couper l'extrémité de préhension (24), ces deux coquilles entourent la partie de cette extrémité de préhension (24) qui doit être détachée et qui se situe au-dessus de l'écrou (28) par rapport à ladite extrémité pénétrante (23).

12. Système selon l'une des revendications 1 à 11, **caractérisé par** le fait que ledit élément coupant comporte un guide monté en coopération avec les deux mâchoires (40, 41), apte à entourer l'extrémité de préhension (24) avant sa coupe.
